# EUROPEAN PATENT APPLICATION

(11) **EP 1 380 840 A1**
(43) Date of publication of application: **14.01.2004**
(21) Application number: 02707178.6
(22) Date of filing: 27.03.2002
(51) Int. Cl.: G01N 33/574

(54) **METHOD OF DIAGNOSING BREAST CANCER**

(30) Priority: 27.03.2001 JP 2001089842
(71) Applicant: TEIKOKU HORMONE MFG. CO., LTD., Tokyo 107-8532 (JP); Tokyo Metropolitan Foundation for Research on Aging and Promotion of Human Welfare, Tokyo 173-0015 (JP)
(72) Inventor: FURUKAWA, Kiyoshi, Kokubunji-shi, Tokyo 185-0004 (JP); GUO, San Chun, Itabashi-ku, Tokyo 173-0013 (JP); SATO, Takeshi, Kawagoe-shi, Saitama 350-1108 (JP)
(74) Representative: Woods, Geoffrey Corlett
(86) International application number: PCT/JP2002/002984
(87) International publication number: WO 2002/077649

(57) **Abstract**

By detecting the GalNAcβ1→4GlcNAcβ1→ structure expressed at the non-reducing termini of Asn-linked sugar chains of glycoproteins in samples prepared from cancer tissues or milk of patients with breast cancer, not only breast cancer but also the grades and malignancy levels of the breast cancers, prognosis of the breast cancer patients after surgery and etc. can be predicted accurately and effectively.

## Description

### TECHNICAL FIELD

The present invention relates to a diagnostic method for breast cancer and a kit for diagnosing breast cancer. More specifically, the present invention relates to a diagnostic method for breast cancer by searching structures of sugar chain moieties of glycoproteins in a sample prepared from human breast tissues, milk, plasma, serum and so on, by detecting the GalNAcβ1→4GlcNAcβ1→ structure expressed at the non-reducing termini of the Asn-linked sugar chains to diagnose breast cancer. According to the diagnostic method of the present invention, not only the diagnosis of the breast cancer but also the grades and malignancy levels of the breast cancers, prognosis of the breast cancer patients after surgery and etc. can be predicted accurately and effectively.

### BACKGROUND ART

It is known that in cancer cells proteins different from those produced by the normal cells are produced, and most of such abnormal proteins are considered to be important substances from a viewpoint of diagnosing tumors, and are so-called tumor markers. As tumor markers, carcinoembryonic antigen (CEA), α-fetoprotein (AFP), human chorionic gonadotropin, and alkaline phosphatase are known. The majority of these tumor markers are glycoproteins. With recent advances in the analytical technologies of sugar chain structures of glycoproteins, researches on sugar chains as tumor markers is being in full bloom these days.

In the case of sugar chains of glycoproteins, there are two types: Asn-linked and mucin-type. In the Asn-linked sugar chains, the GlcNAc residue at the reducing terminal attaches to the amide group of asparagine (Asn) residues of polypeptides via an N-glycosidic linkage. In mucin-type sugar chains, a sugar chain has been attaches to the hydroxy group of serine or threonine residues of polypeptides via an O-glycosidic linkage.

The research on the cancer diagnosis by detection of changes in the sugar chain structures is being actively carried out. As a diagnostic method for cancers by structural analysis of the Asn-linked sugar chains, there has been reported a method in which choriocarcinoma, a malignant chorionic disease, is diagnosed by running a sample prepared from a patient's urine through a *Datura stramonium* agglutinin (DSA) column and then by detecting the presence of a GalNAcβ1→4GlcNAcβ1→ structure expressed at the non-reducing terminal of the Asn-linked sugar chains of the urine glycoproteins based on the affinity to the column (Igaku to Yakugaku, Vol. 26, No. 5, November 1991, 26(5): 973-981) .

As a diagnostic method for cancers based on the structures of mucin-type sugar chains, there has been known a method of diagnosing breast cancer by detecting the GalNAc containing structures of mucin-type sugar chains using a lectin *(Artocarpus integrifiola* agglutinin) isolated from Jack fruit (In Vivo 1989 Jul-Aug; 3(4): 275-278) and the like.

However, a conventional method of diagnosing breast cancer based on the structures of Asn-linked sugar chains of glycoproteins has not been established.

### DISCLOSURE OF THE INVENTION

Thus, it is an object of the present invention to provide a method of diagnosing breast cancer specifically and accurately based on the structures of Asn-linked sugar chains of the glycoproteins and a kit using it.

After intensive and extensive studies to develop a method of specifically diagnosing breast cancer based on the structures of Asn-linked sugar chains of the glycoproteins, the present inventors have analyzed the structures of Asn-linked sugar chains of the glycoproteins from human breast cancer tissues and breast cancer cells, we have found that the GalNAcβ1→4GlcNAcβ1→ structure at the non-reducing terminal present on Asn-linked sugar chains of the glycoproteins in the normal cells decreases in the glycoproteins from the human breast cancer cells, and this phenomenon is highly unique to the breast cancer, which indicates that based on these structures not only the diagnosis of the breast cancer but the estimation of the grades and malignancy levels of the breast cancers and the prognosis of the breast cancer patients after surgery can be performed accurately, and, thereby, have completed the present invention.

Thus, the present invention is a method of diagnosing the breast cancer by analyzing the structures of sugar chains of glycoproteins in samples prepared from human tissues or body fluids, expressing the GalNAcβ1→4GlcNAcβ1→ structure at the non-reducing termini of Asn-linked sugar chains of the glycoproteins to diagnose breast cancer.

Furthermore, the present invention is a kit for diagnosing breast cancer, i.e. a kit comprising reagents for detecting the presence of the GalNAcβ1→4GlcNAcβ1→ structure expressed at the non-reducing termini of Asn-linked sugar chains of the glycoproteins.

### BRIEF EXPLANATION OF THE DRAWINGS

Fig. 1 shows the result by lectin blot analysis of glycoproteins in normal healthy human milk samples.
Fig. 2 shows the result by lectin blot analysis of glycoproteins from patients with breast cancer.
Fig. 3 shows the breast cancer tissues stained with the WFA lectin.
Fig. 4 shows a relationship between the stainability of WFA in the breast cancer tissues and the survival terms of the patients after surgery.

### BEST MODE FOR CARRYING OUT THE INVENTION

Samples for use in the method of diagnosing breast cancer of the present invention are those samples prepared from human tissues and body fluids such as the human breast tissues, milk, plasma, sera, saliva, tears and etc. Particularly preferred are samples from tissues of the human breast or milk. Usually, there can be mentioned samples prepared from tissues of the human breast with cancer suspection, breast cancer tissues of patients actually developing breast cancer, or human milk samples suspected of having breast cancer or patients with breast cancer. Using these samples, proteins may be prepared from the tissues or the milk samples by a conventional method. Alternatively, the tissues or milk can be used as samples as they are.

In order to prepare glycoproteins which are searching molecules in the samples, preparation methods known per se may be used. For example, a surfactant-added buffer may be added to samples to solubilize glycoproteins, and then to solubilized glycoproteins, an appropriate amount of acetone or ammonium sulfate may be added, and after centrifugation, crude-purified glycoproteins can be obtained in the precipitates.

The GalNAcβ1→4GlcNAcβ1→ structure expressed at the non-reducing termini of Asn-linked sugar chains of the glycoproteins in samples can be detected as follows:

It can be carried out by a method in which Asn-linked sugar chains are selectively liberated from proteins in the sample, and using Asn-linked sugar chains obtained, the biding property of the GalNAcβ1→4GlcNAcβ1→ structure is investigated by reagents having an affinity with the structure. In order to liberate Asn-linked sugar chains selectively, chemical treatment, for example, hydrazine, and enzymatically N-glycanase treatment that can excise Asn-linked sugar chains from peptides may be used. Since the GalNAcβ1→4GlcNAcβ1→ structure expressed at the non-reducing termini of sugar chains is not often observed in mucin-type sugar chains but is expressed in Asn-linked sugar chains, glycoproteins can be used for detection as it is without selective liberation of Asn-linked sugar chains from glycoproteins.

As reagents having an affinity with the GalNAcβ1→4GlcNAcβ1→ structure, lectins from a plant wistaria, specifically *Wistaria floribunda* agglutinin (WFA) or lectins having a similar specificity, or a similar affinity with the GalNAcβ1→4GlcNAcβ1→ structure, are preferred. Alternatively, antibodies that can recognize the GalNAcβ1→4GlcNAcβ1→ structure may be used. In the case of the antibody, there can be mentioned antiserum that is obtained by a method in which the GalNAcβ1→4GlcNAcβ1→ moiety is bound to a protein used as a hapten such as bovine serum albumin, which is used as an immunizing antigen, to immunize an animal, from which antiserum is to be obtained. Alternatively, there can be mentioned monoclonal antibodies obtained by a hybridoma constructed by a common cell fusion technique using this immunizing antigen.

In order to actually detect the GalNAcβ1→4GlcNAcβ1→ structure expressed at the non-reducing termini of Asn-linked sugar chains using reagents such as lectin, antibody and etc., for example reagents such as lectin, antibody and etc. are labelled with an enzyme such as peroxidase, and the labelled reagents are to be reacted to glycoproteins or Asn-linked sugar chains liberated therefrom, visualized in a standard method, and the presence of reaction products is to be examined. It is also possible to detect by a conventional dot blot method or a Western blot method using reagents such as lectin, antibody and etc. Furthermore, it is also possible to detect by standard column chromatography using a column in which these reagents are immobilized. Alternatively, detection may be also carried out by histological examination after treatment with these reagents, pathological specimens may be prepared from human breast tissues with suspected breast cancer or breast cancer tissues, which are then stained for examination.

As samples, when samples prepared from human plasma, sera, saliva or tears are used, glycoproteins to be examined are glycoproteins preferably present in both human breast tissues and the milk and human plasma, sera, saliva or tears. When samples prepared from human plasma, sera, saliva or tears are actually used, glycoproteins preferably present in both the human breast tissues and the milk and human plasma, sera, saliva or tears are identified by, for example, an immunoenzymatic method, and then the GalNAcβ1→4GlcNAcβ1→ structure expressed at the non-reducing termini of Asn-linked sugar chains is detected by the method described above.

When the GalNAcβ1→4GlcNAcβ1→ structure at the non-reducing termini of Asn-linked sugar chains is not detected or detected very slightly by the test as above, breast cancer is diagnosed. According to the present invention, in the cell membrane of normal tissues, the sugar chains having the GalNAcβ1→4GlcNAcβ1→ structure at the non-reducing termini are generally present at about 30% of the total Asn-linked sugar chains of the glycoproteins, and Asn-linked sugar chains of the glycoproteins of the breast cancer cells, it decreases to 2-3% of the total. Thus, using these ratios as a rough measurement, the development of breast cancer can be diagnosed. In the case of breast cancer, its grades and malignancy levels also can be diagnosed. Furthermore, the presence of breast cancer and, in the case of breast cancer, its grades and malignancy levels can be diagnosed by performing the detection as described above using samples obtained from human breast tissues with suspected breast cancer or the breast cancer tissues and samples obtained from the normal regions of the tissues extracted from the same patients, and by comparing the results of them. Furthermore, according to the diagnostic method of the present invention, patients with breast cancer after surgery can be prognosed by carrying out the diagnosis of the present invention on patients with breast cancer.

In accordance with the present invention, a kit is also provided for use in the above-mentioned diagnosis. Such a kit contains reagents that detect the presence of GalNAcβ1→4GlcNAcβ1→ structure expressed at the non-reducing termini of Asn-linked sugar chains of glycoproteins. As such reagents, there can be mentioned the above-mentioned lectins, antibodies and the like. In addition to these reagents, reagents such as chromogenic reagents used for the detection method as described above may be added as necessary.

The present invention will now be explained more specifically with reference to the following examples, but it should be noted that the present invention is not limited by these examples in any way.

### Example 1

### Structures of Asn-linked sugar chains of human breast cancer cell line

### (1) Method

Structural analysis of Asn-linked sugar chains of human breast cancer cell line (MRK-nu-1) was performed by the method of Kobata (Kobata, A. and Furukawa, K. (1992) Glycoconjugates: Composition, Structure, and Function (Allen, H.J. and Kisailus, E.C., eds.), pp. 33-69, Marcel Dekker Inc., New York).

Cell membrane proteins of the human breast cancer cell line were prepared, and after releasing with hydrazine the reducing termini of Asn-linked sugar chains were ³H-labelled with NaB³H₄, and analyzed by BioGel P-4 gel filtration chromatography.

### (2) Results

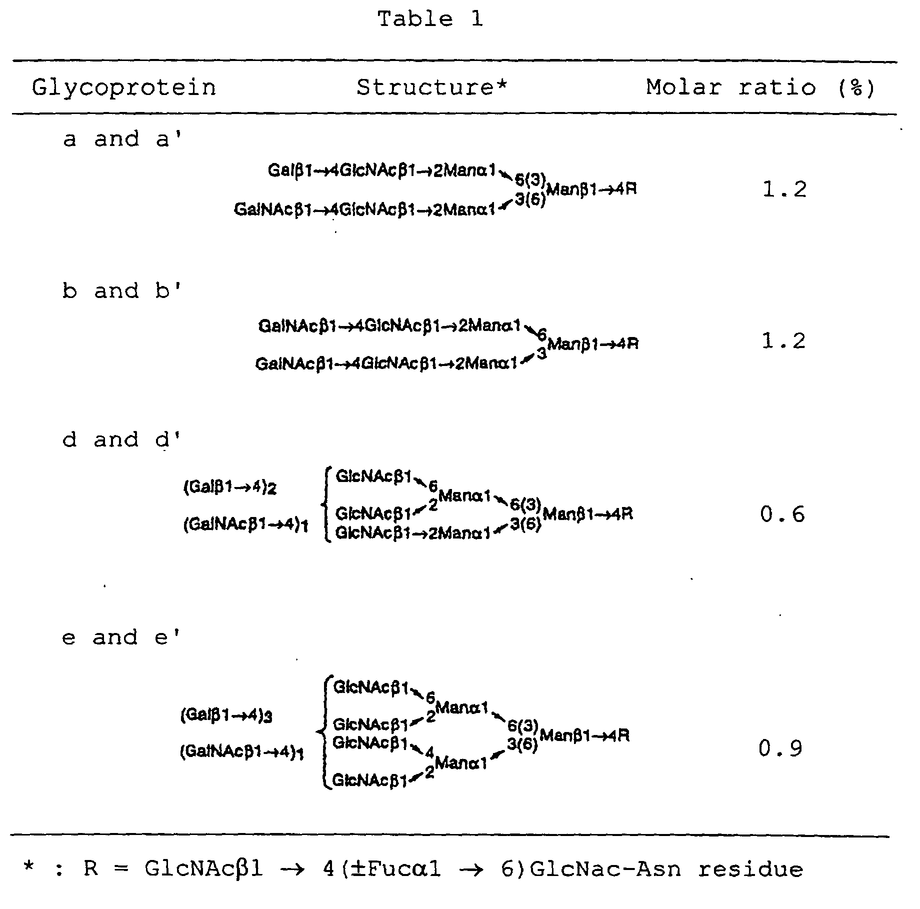

The results obtained are summarized in Table 1.

In Table 1, the column of glycoprotein indicates peaks of sugar chains subjected to gel filtration column chromatography. As can be shown in Table 1, the presence of the GalNAcβ1→4GlcNAcβ1→ structure at the non-reducing termini was confirmed at Asn-linked sugar chains of glycoproteins of the human breast cancer cell line at a very low molar ratio of 3.9%. In the case of normal human cells, the presence of sugar chains having the GalNAcβ1→4GlcNAcβ1→ structure at the non-reducing termini was confirmed at Asn-linked sugar chains at a molar ratio of about 30%.

Based on these, it is clear that in the Asn-linked sugar chains of glycoproteins of breast cancer cells, no or very little GalNAcβ1→4GlcNAcβ1→ structure at the non-reducing termini is detected, and thus by detecting the presence of these structures, breast cancer can be diagnosed.

### Example 2

### Lectin blot analysis of glycoproteins in the milk samples from normal healthy humans

### (1) Method

From two normal healthy humans, milk samples were collected, and treated with acetone to precipitate proteins, which was analyzed by lectin blot (1-5 µg protein/lane). Thus, a lectin derived from wistaria *(Wistaria floribunda* agglutinin: WFA), that binds to the GalNAcβ1→4GlcNAcβ1→ structure, was labelled with peroxidase, which was reacted with glycoproteins to visualize WFA-binding sugar chains. At the same time, glycoprotein sugar chains on the blot were digested with an enzyme (N-acetylhexosaminidase: HexNAc'ase) that decomposes GalNAc, and an enzyme (N-glycanase) that excises the Asn-linked sugar chains from peptides, and then WFA was reacted thereto and visualized.

As a control, a lectin derived from Ricinus communis *(Ricinus communis* agglutinin-I (RCA-I)) was reacted to glycoproteins and visualized similarly. RCA-I is a lectin that binds to sugar chains having the Galβ1→4GlcNAcβ1→ structure. Also, proteins were stained with Coomassie brilliant blue (CBB) and detected.

### (2) Results

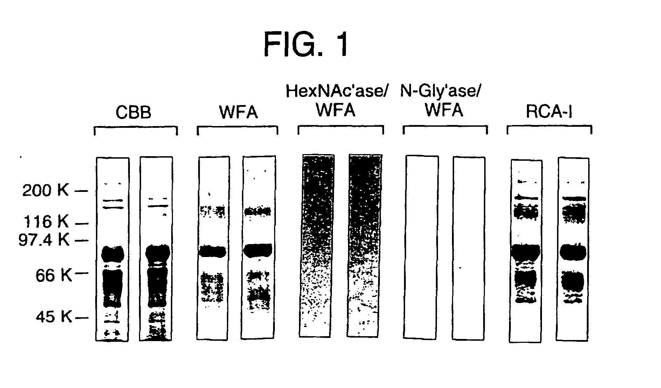

The results obtained are shown in Fig. 1. As can be shown in Fig. 1, when glycoproteins were digested with an enzyme (HexNAc'ase) that decomposes GalNAc, and an enzyme (N-glycanase) that excises Asn-linked sugar chains from peptides, and then reacted with WFA, the GalNAcβ1→4GlcNAcβ1→ structure was not detected. On the other hand, when glycoproteins were not treated with these enzymes, and reacted with WFA as it is, the GalNAcβ1→4GlcNAcβ1→ structure was detected.

Based on these results, it is clear that in glycoproteins from normal healthy human milk samples, the GalNAcβ1→4GlcNAcβ1→ structure is detected in Asn-linked sugar chains.

### Example 3

### Lectin blot analysis of glycoproteins from breast cancer tissues

### (1) Method

From two breast cancer patients (Patient 1 and Patient 2) at stage 1 and two patients (Patient 3 and Patient 4) at stage 2, tissues (1R, 2R, 3R, and 4R, respectively) from the surgically extracted cancer portions and tissues (1L, 2L, 3L, and 4L, respectively) from the noncancer portions were treated with acetone to prepare samples. The samples were subjected to lectin blot analysis as in Example 2.

At the same time, as in Example 2, experiments were performed for samples treated with HexNAc'ase or N-glycanase, and with RCA-I, and with CBB.

### (2) Results

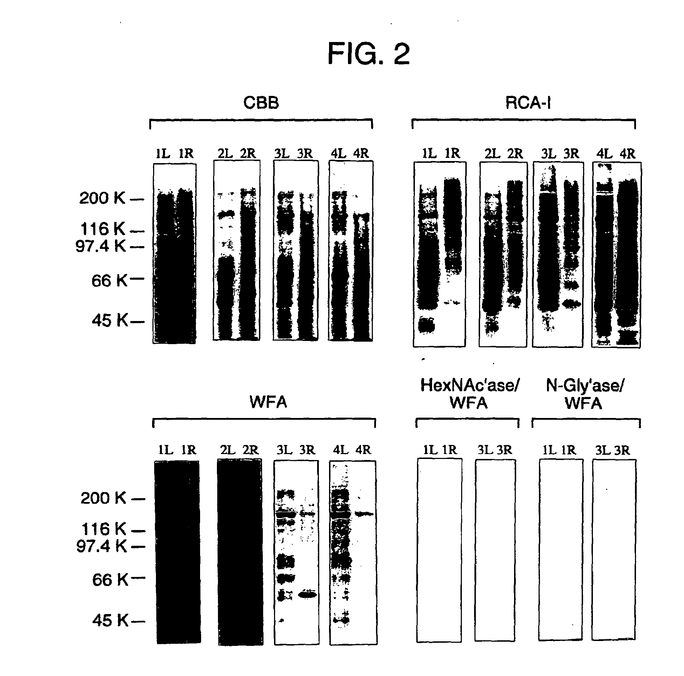

The results obtained are shown in Fig. 2. As can be shown in the results by WFA staining at the bottom left, the GalNAcβ1→4GlcNAcβ1→ structure was detected in glycoproteins of the noncancer portions, whereas the structure was detected very little in glycoproteins of the cancer portions. Furthermore, less GalNAcβ1→4GlcNAcβ1→ structure was detected in glycoproteins from patients at stage 2 than patients at stage 1.

This indicates that by detecting the GalNAcβ1→4GlcNAcβ1→ structure, the grades and malignancy levels of the breast cancers can be diagnosed.

### Example 4

### Histochemistry of the breast cancer tissues stained with WFA

### (1) Method

Tissue sections of cancer tissues at stage 1, stage 2, and stage 3 and benign tumor tissues obtained from patients with breast cancer fixed in paraffin, were incubated with peroxidase-labelled WFA (10 µg/ml), and then visualized by reacting with 3,3'-diaminobenzidine.

### (2) Results

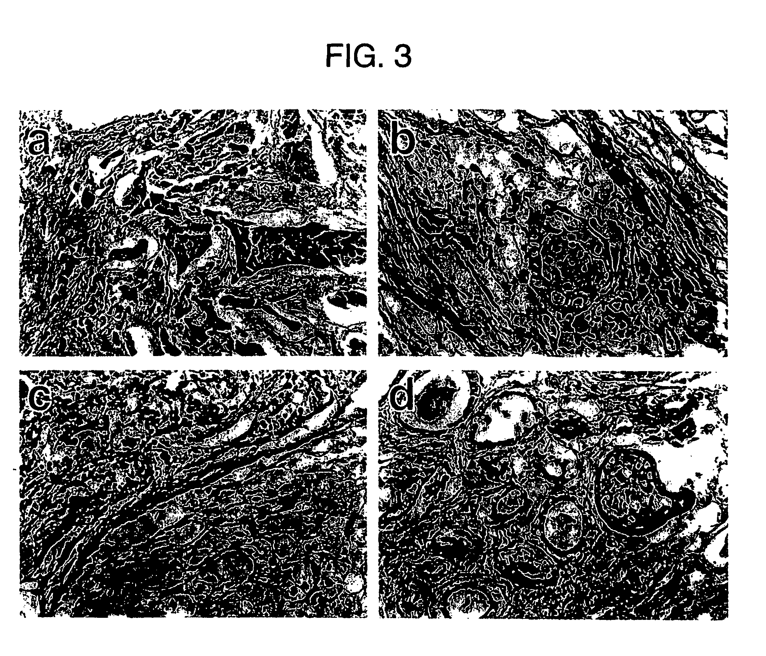

The results obtained are shown in Fig. 3. Panels a, b, c, and d in Fig. 3 represent the results for cancer tissues at stage 1, stage 2, and stage 3 and benign tumor tissues, respectively. In the cancer portions, stainability with WFA decreases in (a) or disappeared in (b, c), and unchanges in the noncancer portion in (d).

### Example 5

### WFA-reactivity of the breast cancer tissues and survival rates of the patients after surgery

### (1) Method

Cancer tissues (for pathology) taken from patients with breast cancer at a hospital were stained in a manner similar to Example 4, and its relationship with survival rates of the patients after surgery was analyzed.

### (2) Results

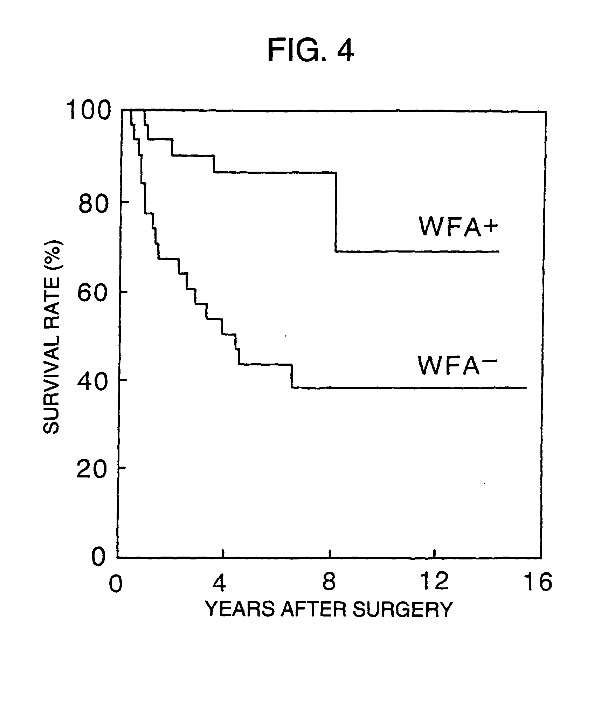

The results obtained is shown in Fig. 4. In Fig. 4, WFA⁺ represents survival rates of 31 patients at stage 1 after surgery whose reactivity with WFA is presented, and WFA⁻ represents survival rates of 30 patients with breast cancer at stage 2 or 3 after surgery (whose reactivity with WFA is decreased or disappeared). It can be noticed that the 10-year survival rate is 70% in WFA⁺ group and 38% in WFA⁻ group.

Based on these results, it can be seen that prognosis of patients with breast cancer after surgery is diagnosed by the diagnostic method of the present invention.

### Industrial Applicability

As described in detail above, by the diagnostic method of the present invention, the grades and malignancy levels of the breast cancers, prognosis of breast cancer patients after surgery etc. as well as the presence or absence of breast cancer can be predicted accurately and effectively.

## Claims

1. A method of diagnosing breast cancers by analyzing the structures of sugar chains of glycoproteins in samples prepared from human tissues or body fluids, comprising detecting the presence of the GalNAcβ1→4GlcNAcβ1→ structure expressed at the non-reducing termini of Asn-linked sugar chains of glycoproteins to diagnose breast cancer.

2. The method of diagnosing breast cancer according to claim 1 wherein breast cancers are diagnosed when the GalNAcβ1→4GlcNAcβ1→ structure is not detected or detected very little at the non-reducing termini of Asn-linked sugar chains.

3. The method of diagnosing breast cancer according to claim 1 or 2 wherein the grades and malignancy levels of the breast cancers are diagnosed by the degree of detecting the GalNAcβ1→4GlcNAcβ1→ structure expressed at the non-reducing termini of Asn-linked sugar chains.

4. The method of diagnosing breast cancer according to claim 1 wherein prognosis of breast cancers after surgery can be diagnosed by the degree of detecting the GalNAcβ1→4GlcNAcβ1→ structure expressed at the non-reducing termini of Asn-linked sugar chains of glycoproteins in samples prepared from tissues or body fluids of patients with breast cancer.

5. The method of diagnosing breast cancer according to any of claims 1 to 4 wherein the samples are samples prepared from human breast tissues or milk.

6. The method of diagnosing breast cancer according to any of claims 1 to 5 comprising detecting the GalNAcβ1→4GlcNAcβ1→ structure expressed at the non-reducing termini of Asn-linked sugar chains by affinity of lectins.

7. The method of diagnosing breast cancer according to any of claims 1 to 5 comprising detecting the GalNAcβ1→t4GlcNAcβ1→ structure expressed at the non-reducing termini of Asn-linked sugar chains by antibodies that can recognize the GalNAcβ1→4GlcNAcβ1→ structure.

8. A kit for diagnosing breast cancers said kit comprising reagents for detecting the GalNAcβ1→4GlcNAcβ1→ structure expressed at the non-reducing termini of Asn-linked sugar chains of glycoproteins.

9. The kit according to claim 8 wherein the reagents are lectins.

10. The kit according to claim 9 wherein the lectin is *Wistaria floribunda* agglutinin or a lectin having a similar specificity thereto.

11. The kit according to claim 8 wherein the reagent is an antibody that can recognize the GalNAcβ1→4GlcNAcβ1→ structure.
